Europäisches Patentamt

European Patent Office

Office européen des brevets

(19)

(11) **EP 0 632 920 B1**

## (12) FASCICULE DE BREVET EUROPEEN

(45) Date de publication et mention
de la délivrance du brevet:
**07.04.1999 Bulletin 1999/14**

(21) Numéro de dépôt: **94905137.9**

(22) Date de dépôt: **21.01.1994**

(51) Int Cl.⁶: $G10K\ 11/34$

(86) Numéro de dépôt international:
**PCT/FR94/00070**

(87) Numéro de publication internationale:
**WO 94/17514 (04.08.1994 Gazette 1994/18)**

(54) **APPAREIL DE THERAPIE A FOCALISATION VARIABLE SANS FOCALISATION SECONDAIRE**

THERAPIEGERÄT MIT VARIABLER FOKUSSIERUNG OHNE SEKUNDÄRFOKUSSIERUNG

THERAPY APPARATUS WITH VARIABLE FOCALIZATION WITHOUT SECONDARY FOCALIZATION

(84) Etats contractants désignés:
**DE DK FR GB IT SE**

(30) Priorité: **22.01.1993 FR 9300662**

(43) Date de publication de la demande:
**11.01.1995 Bulletin 1995/02**

(60) Demande divisionnaire: **97200222.4**

(73) Titulaires:
• **TECHNOMED MEDICAL SYSTEMS**
**69500 Bron (FR)**
• **INSTITUT NATIONAL DE LA SANTE ET DE LA RECHERCHE MEDICALE (INSERM)**
**75654 Paris Cédex 13 (FR)**

(72) Inventeurs:
• **CHAPELON, Jean-Yves**
**F-69100 Villeurbanne (FR)**
• **CATHIGNOL, Dominique**
**F-69740 Genas (FR)**
• **BLANC, Emmanuel**
**F-69230 St.-Genis-Laval (FR)**

(74) Mandataire: **Hirsch, Marc-Roger et al**
**Cabinet Hirsch**
**34 rue de Bassano**
**75008 Paris (FR)**

(56) Documents cités:
• **IEEE TRANSACTIONS ON ULTRASONICS, FERROELECTRICS, AND FREQUENCY CONTROL, vol.38, no.5, Septembre 1991, NEW YORK, USA pages 510 - 520, XP230197 EBBINI ET AL 'Experimental evaluation of a prototype cylindrical section ultrasound hyperthermia phased-array applicator' cité dans la demande**
• **IEEE TRANSACTIONS ON ULTRASONICS, FERROELECTRICS, AND FREQUENCY CONTROL, vol.36, no.2, Mars 1989, NEW YORK, USA pages 249 - 257 UMEMURA ET AL 'The sector-vortex phased arry : acoustic field synthesis for hyperthermia' cité dans la demande**
• **IEEE TRANSACTIONS ON ULTRASONICS, FERROELECTRICS, AND FREQUENCY CONTROL, vol.39, no.3, Mai 1992, NEW YORK, USA pages 341 - 351, XP289695 O'DONNELL 'Coded excitation system for improving the penetration of real-time phased-array imaging systems'**

## Description

La présente invention concerne essentiellement un appareil de thérapie à focalisation variable sans focalisation secondaire. De préférence, l'appareil de thérapie est un appareil de thérapie par ultrasons focalisés. L'invention concerne aussi de manière générale l'utilisation d'un signal électronique à fonction d'autocorrélation de type DIRAC pour la commande d'au moins un élément transducteur ultrasonique de thérapie ; et un procédé de focalisation dynamique électronique d'au moins un élément transducteur ultrasonique de thérapie éliminant ou minimisant la focalisation secondaire grâce à l'utilisation d'un générateur de signal délivrant un signal électronique dont la fonction d'autocorrélation est de type Dirac.

On sait que la thérapie par ultrasons focalisés, à l'aide de transducteurs piézoélectriques activés par des signaux électroniques de type sinusoïdal, permet de créer des lésions tissulaires qui sont limitées au point focal du transducteur ultrasonique ce qui est particulièrement nécessaire pour un traitement efficace dans le cadre des thérapies du cancer comme par exemple de la prostate, du sein, du cerveau, etc.

Généralement, ces cancers présentent un certain volume et de ce fait, pour traiter la totalité du volume d'une lésion, il est nécessaire de déplacer le point focal du transducteur pour lui faire parcourir la totalité du volume à détruire.

Ce déplacement peut être obtenu par des moyens mécaniques, généralement des dispositifs de motorisation qui sont encombrants et coûteux. On peut aussi utiliser des moyens électroniques de déplacement du point focal qui offrent les avantages d'être moins encombrants, d'être moins coûteux, de supprimer tout mouvement mécanique du transducteur sachant que ces mouvements perturbent le contact entre le transducteur et le patient et nuisent à la bonne transmission des ondes acoustiques et qu'ils risquent encore de provoquer un déplacement de la zone cible en cours de traitement. On peut aussi disposer et immobiliser les transducteurs à focalisation électronique au contact de la peau des patients, puis tirer d'abord en profondeur en utilisant la focale électronique maximale et ensuite en surface en utilisant la focale minimale. On sait qu'un des risques de cette méthode de traitement consiste en des brûlures de la peau des patients qui sont diminuées lorsque l'ouverture est maximale. L'ouverture est le rapport entre le diamètre du faisceau et la distance focale, donc est minimale pour la focale électronique la plus grande et maximale pour la plus petite focale électronique. On conçoit que la méthode décrite ci-dessus minimise les risques de brûlure puisque les tirs en surface sont effectués à ouverture maximale.

Pour utiliser ces moyens électroniques de déplacement du point focal il est nécessaire d'avoir recours à des transducteurs à structure multi-éléments. Ces multi-éléments peuvent être de forme annulaire pour obtenir un déplacement du point focal dans la direction de l'axe de propagation acoustique.

On peut également utiliser un transducteur à structure multi-éléments de forme mosaïque pour obtenir un déplacement du point focal dans les trois directions de l'espace.

Le déplacement physique du point focal est alors obtenu en appliquant à chaque élément du transducteur des signaux électroniques de type sinusoïdal déphasés les uns par rapport aux autres.

Il est connu par l'homme de l'art que pour des amplitudes importantes de déplacement du point focal il peut apparaître une ambiguïté sur les valeurs de déphasage affectées aux signaux de commandes de chacun des éléments du transducteur. Cette ambiguïté de phase fait alors apparaître un point de focalisation secondaire dans le champ ultrasonore du transducteur. Ce point de focalisation secondaire entraîne une nécrose tissulaire dans une zone qui n'est pas ciblée et condamne ainsi l'utilisation de transducteurs à focalisation dynamique pour des raisons de sécurité thérapeutiques évidentes.

Pour répondre à ce problème, on a proposé diverses solutions qui permettent de contrôler le champ ultrasonore et d'obtenir un champ ultrasonore particulier préalablement établi et qui élimine les points de focalisation secondaires.

On peut citer à ce sujet en particulier la méthode de synthèse de faisceaux décrite par exemple par E.S. EBBINI dans l'article "Experimental evaluation of a prototype cylindrical section ultrasound hyperthermia phased-array applicator" (dans IEEE transactions on ultrasonics, ferroelectrics, and frequency control. vol. 38 n° 5 p 510 à 520, September 1991). Cette méthode consiste à déterminer les valeurs d'amplitude et de phase sur chaque élément du transducteur qui permettent de former une géométrie donnée du faisceau. Un exemple est donné sur les figures 6, 9, 10 et 11 de ce document, et EBBINI montre qu'avec cette méthode il est possible de synthétiser un champ ultrasonore à un ou plusieurs, en particulier 3, points de focalisation.

Cette méthode présente un inconvénient majeur. Chaque transducteur n'est pas alimenté par le signal d'amplitude maximale qu'il pourrait recevoir (voir en particulier p 514). L'énergie acoustique générée n'est alors pas maximale, ce qui est totalement incompatible avec un traitement endocavitaire par ultrasons de forte puissance où le rapport puissance émise sur dimension du transducteur doit être maximal. Par contre, cette contrainte est acceptable pour un traitement du type hyperthermie comme envisagé dans cet article où les puissances émises sont largement plus faibles (voir p 514).

Une autre méthode de synthèse de faisceaux pour limiter les points de focalisation secondaire est décrite par UMEMURA dans l'article "the Sector-Vortex Phased Array : Acoustic Field Synthesis for Hyperthermia" également dans la revue IEEE transactions on ultrasonics, ferroelectrics, and frequency control. vol. 36, n° 2, p 249 à 257, mars 1989. Cette méthode s'applique à des transducteurs divisés en plusieurs secteurs et consiste à appliquer une distribution de phase particulière sur chaque secteur. Comme cette méthode consiste à subdiviser les anneaux en secteurs, on aboutit à un nombre élevé d'éléments. Pour obtenir une forme de faisceau convenable, le nombre d'éléments doit être élevé, chaque circuit électronique de commande étant complexe, le coût d'un tel dispositif est donc très important, ce qui est difficilement compatible avec une exploitation industrielle et médicale surtout avec la nécessité de limiter les coûts de traitement thérapeutique.

La présente invention a donc pour but principal de résoudre le problème technique de la focalisation dynamique de transducteur de thérapie sur des distances de balayage du faisceau importantes, en éliminant la focalisation secondaire, en utilisant les transducteurs à leur puissance maximale, en conservant une tâche focale de forme ponctuelle, et à un coût permettant une large diffusion industrielle et médicale.

La présente invention apporte pour la première fois une solution satisfaisante à ce problème technique ainsi défini et présente d'autres avantages techniques qui apparaîtront clairement à l'homme de l'art à partir de la description détaillée qui va suivre de l'invention.

Ainsi, selon un premier aspect, la présente invention fournit un procédé de focalisation dynamique électronique d'au moins un élément transducteur piézoélectrique ultrasonore de thérapie, éliminant ou minimisant la focalisation secondaire, comprenant l'activation de l'élément transducteur de thérapie par un signal électronique délivré par un générateur de signal, caractérisé en ce qu'on utilise un générateur de signal délivrant un signal électronique dont la fonction d'autocorrélation est de type DIRAC. La définition de la fonction d'autocorrélation est la suivante :

soit X(t) un signal quelconque, il transporte une certaine énergie :

$$E_x = \int [X(t)]^2 dt$$

La fonction d'intercorrélation entre deux signaux X(t) et Y(t) est définie par :

$$\Gamma_{xy}(\tau) = \int X(t) Y(t - \tau) dt$$

Cette relation chiffre en fonction d'un décalage temporel $\tau$ la ressemblance entre le signal X(t) et le signal Y(t) retardé de cette valeur $\tau$.

Si cette fonction est nulle ($\Gamma_{xy}$=O) on dit alors que les deux signaux ne sont pas corrélés.

De même, on définit la fonction autocorrélation dans le cas où y(t)=x(t) ; on a alors :

$$\Gamma_{xx}(\tau) = \int X(t) X(t - \tau) dt$$

Cette fonction $\Gamma_{xx}(\tau)$ représente donc la ressemblance entre une fonction à l'instant t et cette même fonction a l'instant t-$\tau$. Plus cette ressemblance est faible plus cette fonction se rapproche de 0 tout en gardant son maximum pour $\tau$=O. En effet, quel que soit le type de signal X(t), la fonction d'autocorrélation à sa valeur maximale pour t=0 puisque $\Gamma_{xx}(0)$ n'est autre que l'énergie $E_x$ de ce signal.

Une catégorie de signaux particulièrement intéressante correspond à celle des signaux large bande. Un signal est dit à large bande lorsque sa fonction d'autocorrélation a une largeur $\theta$ très étroite qui tend théoriquement vers une impulsion de DIRAC $\delta(t)$. Nous appellerons un tel signal dans le cadre de la présente description et des revendications, un signal à fonction d'autocorrélation de type DIRAC.

Comme exemple connu de signaux à fonction d'autocorrélation de type DIRAC, on peut citer en particulier :

- les signaux aléatoires de type Gaussien ou Poissonien,
- les signaux à modulation de fréquence ou de phase.

On peut encore citer les signaux pseudo-aléatoires codés parmi lesquels :

- les signaux à séquence "M" ou encore dénommés séquences binaires à longueur maximale du type de ceux qui sont décrits au chapitre 6 par Jean-Yves Chapelon, p 225 à 236, en particulier à partir de la p 230 du livre "Progress in medical imaging", édité par le professeur Newhouse éditeur Springer Verlag, New York, année 1988.
- les codes de "GOLAY"
- les codes de "BARKER".

Les signaux pseudo-aléatoires codés peuvent être utilisés directement ou peuvent moduler en phase ou en fréquence un signal électronique dont la fréquence porteuse correspond à la fréquence nominale de fonctionnement du transducteur.

On préfère en particulier les signaux codés de type pseudo-aléatoire de séquence "M". Ces signaux sont décrits précisément dans "Progress in medical imaging". Il s'agit succinctement de séquences de signaux binaires construits par la répétition pseudo-aléatoire d'impulsions ou "pulses" d'une durée élémentaire. Chacune de ces séquences se répète avec une période de répétition "T" caractéristique de la séquence "M".

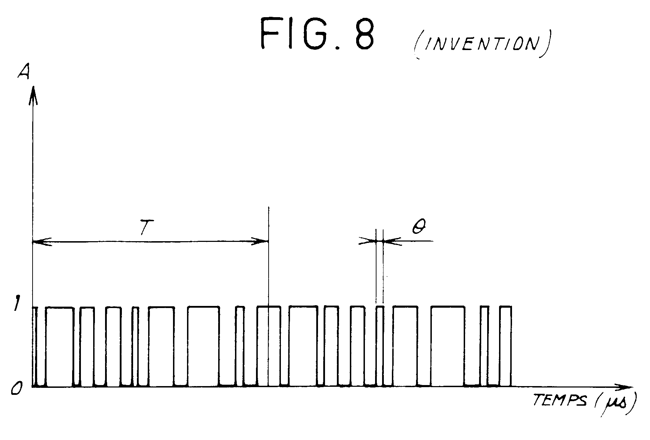

On peut donner une description plus précise d'un signal de séquence "M" en référence à la figure 8 annexée :

- durée d'impulsion ou "pulse" élémentaire "θ" : 0,1 μs < θ < 100 μs, idéalement de valeur 2 à 10 fois la période d'oscillation du transducteur,
- période de répétition "T" : 1 μs < T < 10 s.

Les signaux codés de type pseudo-aléatoire, en particulier de type pseudo-aléatoire de séquence "M" actuellement préféré, sont réalisables aisément à partir de circuits électroniques bien connus de l'homme de l'art.

Grâce à ces signaux électroniques à fonction d'autocorrélation de type DIRAC, il est possible d'éliminer toute ambiguïté dans la définition des retards, ce qui aboutit à une focalisation en un point unique en éliminant ainsi, de manière fiable et sûre, les zones focales secondaires antérieurement existantes.

Selon un deuxième aspect, la présente invention fournit également un appareil de thérapie à focalisation dynamique électronique, comprenant un dispositif ultrasonore de thérapie comprenant au moins un élément transducteur ultrasonore de thérapie, un générateur de signal délivrant un signal électronique ainsi qu'un dispositif de commande d'un tel générateur de signal, caractérisé en ce que le générateur de signal délivre un signal électronique dont la fonction d'autocorrélation est de type DIRAC.

Les modes de réalisations préférés de ce signal ou de l'appareil résultent de la description précédente relative au procédé ainsi que des revendications.

Dans l'un ou l'autre des aspects précédents, selon un mode de réalisation avantageux, le générateur de signal délivre un signal binaire à fonction d'autocorrélation du type DIRAC du type "séquence M" en particulier dit "à longueur maximale".

D'autres caractéristiques apparaîtront également à l'homme de l'art à partir de la description suivante, ainsi que des revendications qui font également partie de la présente description.

L'invention va maintenant être décrite avec un mode de réalisation actuellement préféré donné simplement à titre d'illustration qui ne saurait donc en aucune façon limiter la portée de l'invention, en référence aux dessins annexés dans lesquels :

- la figure 1 représente un schéma de principe général d'un appareil de thérapie connu pour réaliser une thérapie des tissus d'un être vivant, comprenant un dispositif multitransducteur ayant des transducteurs piézoélectriques, ici selon un arrangement annulaire de la surface émettrice permettant une focalisation dynamique dans l'axe de propagation ;

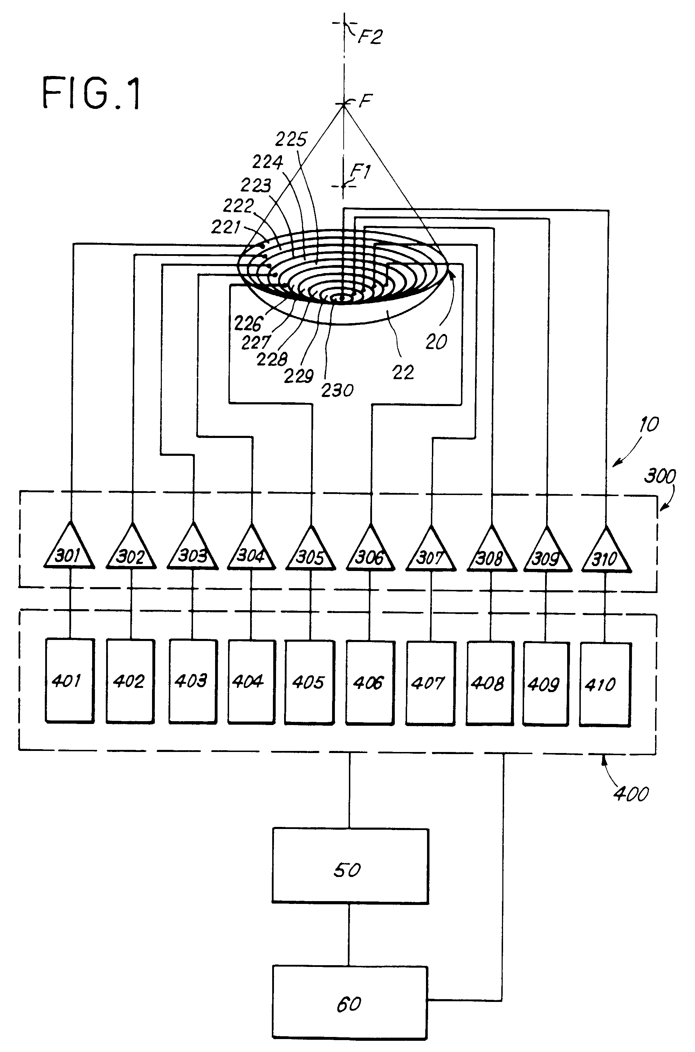

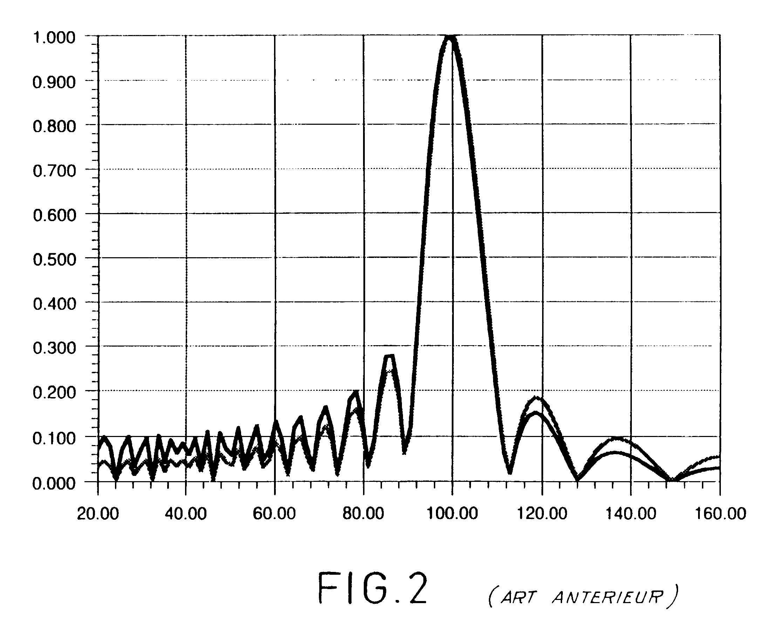

- la figure 2 représente une courbe de pression ultrasonique exprimée en bars, ici normalisée à 1 en ordonnées, en fonction de la distance axiale définie par l'axe de propagation des ondes ultrasoniques, en abscisse, exprimée en millimètres de l'élément transducteur de la figure 1, de diamètre 100 mm et à focalisation naturelle au point focal F1 disposé à 100 mm, la courbe en traits grisés est celle qui est obtenue dans le milieu de couplage tel que l'eau, c'est-à-dire sans absorption acoustique ; et la courbe en traits forts est celle qui est obtenue dans un milieu dont les caractéristiques acoustiques sont similaires à celles des tissus d'un être vivant, qui présente une absorption acoustique de 0,1 Neper/cm avec un signal électronique sinusoïdal sans déphasage selon l'art antérieur ;
- la figure 3 est une courbe similaire à celle de la figure 2 obtenue avec le même élément transducteur de la figure 1, (à distance focale naturelle de 100 mm), avec un signal électronique classique de type sinusoïdal déphasé selon les anneaux de manière à obtenir une focalisation à la distance focale de 50 mm ;
- la figure 4 est une courbe similaire à celle des figures 2 et 3 obtenue avec un signal électronique sinusoïdal déphasé, selon l'art antérieur, de manière à obtenir une focalisation à la distance focale de 130 mm ;
- la figure 5 représente une courbe similaire à celle des figures 2 à 4, obtenue avec un signal électronique de type pseudo-aléatoire à séquence M, selon la présente invention, sans déphasage entre les transducteurs en obtenant ainsi la focalisation naturelle à 100 mm ;
- la figure 6 représente une courbe similaire à la figure 5 avec le même signal pseudo-aléatoire à séquence M selon la présente invention mais avec des retards entre anneaux permettant d'obtenir une focalisation électronique à 50 mm ;
- la figure 7 représente une courbe similaire aux figures 5 et 6 avec le même signal pseudo-aléatoire à séquence M selon la présente invention avec des retards entre anneaux permettant d'obtenir une focalisation à 130 mm, et

- la figure 8 représente un signal binaire codé de type pseudo-aléatoire selon l'invention de séquence M, dont la description plus précise a été précédemment donnée en page 5, avec en ordonnée l'amplitude normalisée à 1 et en abscisse le temps en microsecondes.

En référence à la figure 1, on a représenté schématiquement un appareil de thérapie connu représenté par le numéro de référence général 10 pour réaliser la thérapie de tissus d'un être vivant.

Cet appareil de thérapie 10 comprend un dispositif de thérapie proprement dit 20 ici sous forme de calotte sphérique 22 à focalisation naturelle, subdivisée en réseau annulaire tel que représenté, ou en mosaïque (non représenté) d'éléments transducteurs piézoélectriques tels que 221 à 230. Ce réseau annulaire 221 à 230 ou en mosaïque (non représenté) est bien connu à l'homme de l'art et aucune description complémentaire n'apparaît nécessaire. Un exemple de réalisation d'un tel dispositif de thérapie est par exemple sous forme de calotte sphérique 22 de diamètre 100 mm à focalisation naturelle à la distance focale de 100 mm, ayant une fréquence de fonctionnement à 1 Mhz environ, et une structure annulaire de dix anneaux ici de surface constante séparés chacun par un espace de 0,1 mm, représentée à la figure 1.

Chaque élément transducteur annulaire 221 à 230 est relié via un dispositif amplificateur 300 comprenant des amplificateurs individuels 301 à 310 et un dispositif de lignes à retard 400 comprenant des lignes individuelles à retard 401 à 410 à un générateur de signaux ici commun 50, lui-même commandé par une centrale de commande 60. La centrale de commande 60 commande également les lignes à retard 401 à 410 en fournissant la valeur de retard nécessaire à chaque ligne pour obtenir une focalisation à la distance de focalisation recherchée.

Grâce à cette conception de l'appareil, il est possible de réaliser une focalisation dynamique électronique, par exemple entre les points focaux F1 et F2.

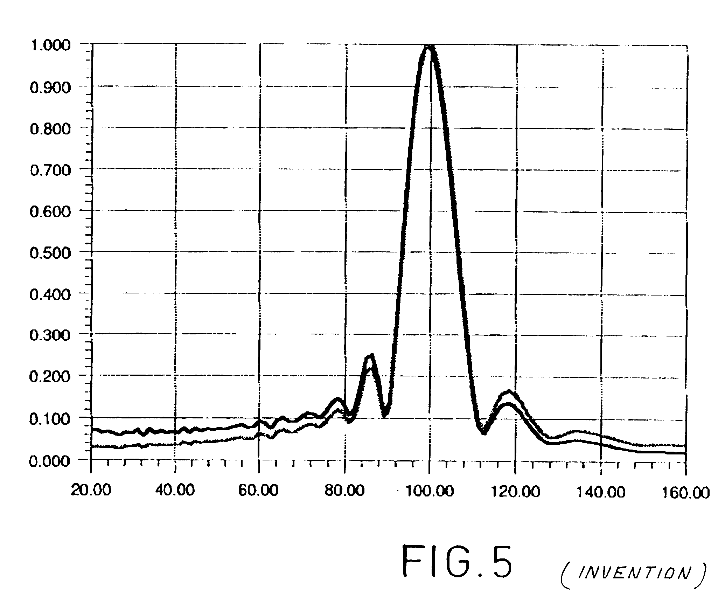

Le fonctionnement de cet appareil va maintenant être décrit en référence aux courbes des figures 2 à 7, respectivement selon l'art antérieur (figures 2 à 4) et selon l'invention (figures 5 à 8).

Tout d'abord, lorsque l'on fait générer par le générateur 50 un signal électronique classique de type sinusoïdal, à une fréquence d'environ 1 MHz, et lorsqu'on ne commande aucun retard aux lignes à retard 401 à 410, on obtient une focalisation naturelle au point focal géométrique F1, ici à 100 mm, en obtenant ainsi la courbe de la figure 2.

Lorsque, grâce à la commande 60, on envoie le même signal par le générateur 50 mais en introduisant des retards entre les anneaux dans les lignes 401 à 410, on peut déplacer le point focal le long de l'axe.

Si on utilise les retards suivants exprimés en µs, le point focal sera déplacé respectivement à 50 mm ou à 130 mm :

| Anneau n° | Point focal à 50 mm, retard en µs | Point focal à 130 mm, retard en µs |
|-----------|-----------------------------------|------------------------------------|
| 1 | 0 | 0 |
| 2 | 1,2 | - 0,3 |
| 3 | 2,4 | - 0,6 |
| 4 | 3,5 | - 0,9 |
| 5 | 4,6 | - 1,2 |
| 6 | 5,7 | - 1,5 |
| 7 | 6,7 | - 1,8 |
| 8 | 7,8 | - 2 |
| 9 | 8,8 | - 2,3 |
| 10 | 9,8 | - 2,7 |

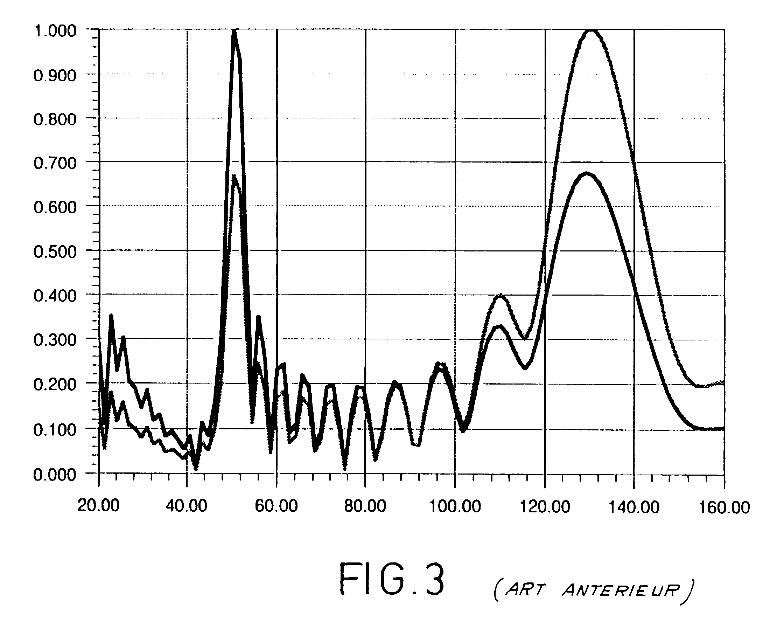

Lorsqu'on utilise les valeurs de retard pour obtenir une focalisation électronique au point focal situé à 50 mm, on obtient les courbes correspondantes représentées à la figure 3. On observe dans ce cas sur les courbes de la figure 3 l'apparition d'une zone focale secondaire entre 120 et 130 mm qui perturbe la zone focale recherchée à 50 mm. On remarquera ici que la zone focale secondaire à 120-130 mm est fortement atténuée en raison de l'atténuation forte des tissus de l'être vivant et a de ce fait une faible incidence sur le traitement. Si on ne prend pas en compte l'atténuation des tissus, la zone focale secondaire a une amplitude plus forte que la zone focale principale à 50 mm, ce qui démontre le risque de l'existence d'une zone focale secondaire en pratique généralement incompatible avec un traitement thérapeutique en toute sécurité.

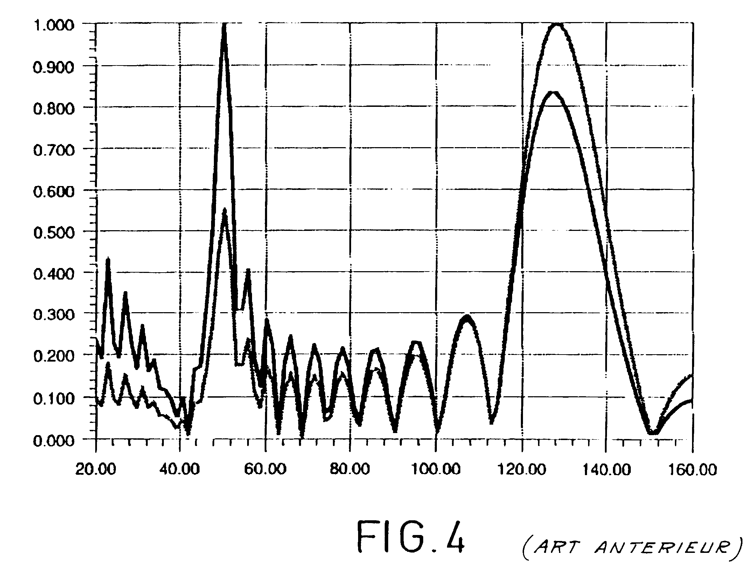

Par contre, lorsque l'on utilise les valeurs de retard permettant de réaliser une focalisation électronique à une distance de l'ordre de 130 mm, on obtient les courbes représentées à la figure 4. A la figure 4, on observe une zone focale secondaire vers 50 mm qui devient une zone focale principale par rapport à la zone focale recherchée à 130 mm qui devient pratiquement inexistante, avec l'effet d'absorption acoustique (courbe en traits forts). Il en résulte une impossibilité de réalisation d'un traitement thérapeutique à cette distance focale.

On observe ainsi qu'à l'aide de signaux électroniques de type classique sinusoïdaux, il est impossible d'avoir une grande variation de distance focale, la focalisation dynamique étant ainsi grandement limitée, ce qui limite d'autant l'attrait de la focalisation dynamique électronique. D'autre part, comme on l'observe à partir de la figure 4, dans certains cas il est impossible d'effectuer un traitement tel qu'il était prévu à une distance focale de 130 mm.

En référence aux figures 5 à 7 réalisées à l'aide du procédé et de l'appareil selon l'invention, on voit l'intérêt majeur de l'utilisation de l'invention en mettant en oeuvre des signaux électroniques à fonction d'autocorrélation de type DIRAC, comme par exemple les signaux électroniques pseudo-aléatoires à séquence M représentés à la figure 8.

En effet, on observe à la figure 5 comparativement à la figure 2 que le signal à fonction d'autocorrélation de type DIRAC donne les mêmes résultats bénéfiques qu'un signal de type sinusoïdal lors d'une focalisation naturelle au point focal géométrique F1, par exemple ici à 100 mm. Ainsi, l'utilisation de signaux à fonction d'autocorrélation de type DIRAC est similaire à celle de signaux sinusoïdaux, ce qui confirme leur possibilité d'utilisation dans une application thérapeutique.

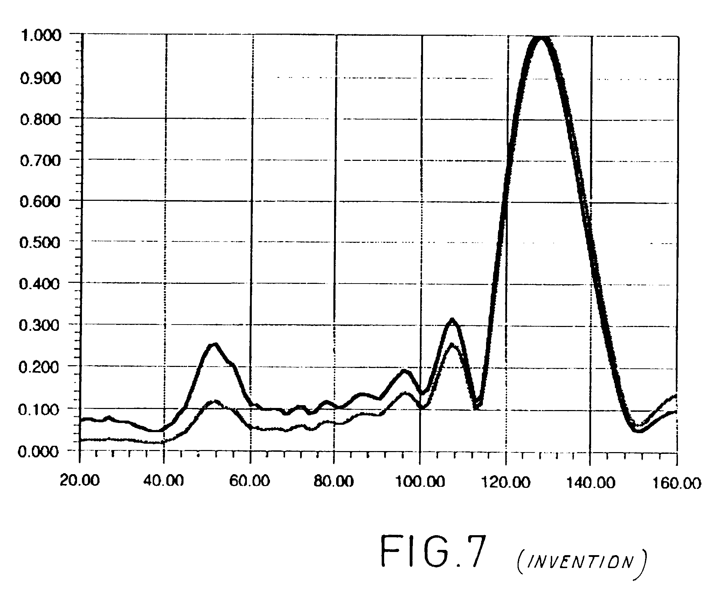

Lorsque l'on recherche à déplacer le point focal par l'introduction de retards comme indiqué précédemment, pour obtenir des distances focales très différentes du point focal géométrique naturel, on observe par exemple en comparant la figure 6 à la figure 3 pour une focalisation électronique à la distance focale de 50 mm avec les mêmes valeurs de retard qu'indiquées au tableau précédents que l'on maintient une zone focale unique contrairement à ce qui a été obtenu avec des signaux électroniques sinusoïdaux classiques.

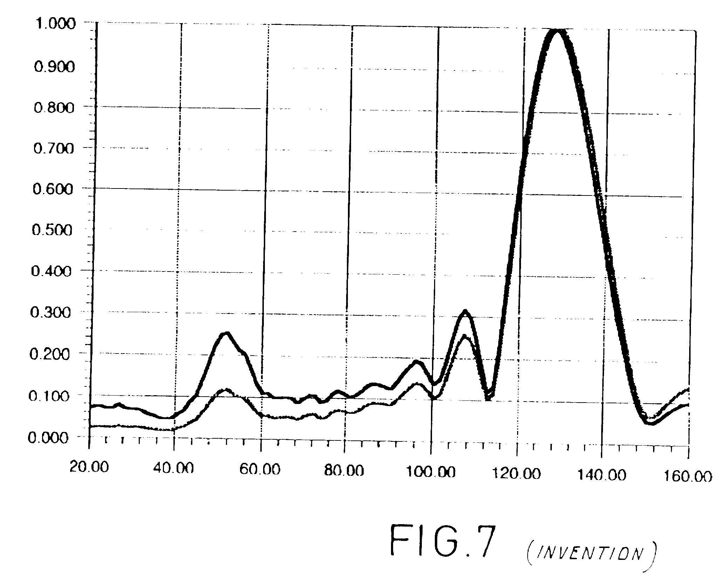

Le même phénomène est observé en comparant la courbe de la figure 7 obtenue avec le signal électronique pseudo-aléatoire à séquence M selon l'invention par rapport à la courbe de la figure 4 obtenue avec un signal électronique sinusoïdal classique. Avec l'invention, une zone focale unique est obtenue contrairement à ce qui est obtenu avec les signaux électroniques sinusoïdaux classiques.

L'invention permet donc d'obtenir un traitement thérapeutique des tissus d'un être vivant d'une manière extrêmement fiable et sûre, efficace dans une grande variété de distances focales, ce qui était pratiquement impossible avec les signaux électroniques sinusoïdaux antérieurs en raison de l'existence de zones focales secondaires apparaissant en dehors du point focal géométrique.

Naturellement, l'invention comprend tous les moyens constituants des équivalents techniques des moyens décrits ainsi que leurs diverses combinaisons. En outre, les dessins font partie intégrante de l'invention et donc de la présente description. L'invention couvre en outre toute caractéristique qui apparaît être nouvelle vis-à-vis d'un état de la technique quelconque et qui résulte de la description, incluant les revendications et les figures, prise dans son ensemble.

## Revendications

1. Procédé de focalisation dynamique électronique d'au moins un élément transducteur piézoélectrique ultrasonore de thérapie (221-230), éliminant ou minimisant la focalisation secondaire, comprenant l'activation de l'élément transducteur ultrasonore par un signal électronique délivré par un générateur de signal, caractérisé en ce qu'on utilise un générateur de signal (50) délivrant un signal électronique dont la fonction d'autocorrélation est de type DIRAC.

2. Procédé selon la revendication 1, caractérisé en ce que le générateur de signal (50) délivre un signal électronique à fonction d'autocorrélation de type DIRAC qui est utilisé pour moduler en phase ou en fréquence un signal électronique dont la fréquence porteuse correspond à la fréquence nominale de fonctionnement de l'élément transducteur.

3. Procédé selon la revendication 1 ou 2, caractérisé en ce que le générateur de signal délivre un signal électronique de type binaire à fonction d'autocorrélation de type DIRAC.

4. Procédé selon la revendication 1, 2 ou 3, caractérisé en ce que le générateur de signal délivre un signal électronique aléatoire de type Gaussien ou Poissonien.

5. Procédé selon la revendication 1, 2 ou 3, caractérisé en ce que le générateur de signal délivre un signal électronique à modulation de fréquence ou de phase.

6. Procédé selon la revendication 1, 2 ou 3, caractérisé en ce que le générateur de signal délivre un signal électronique pseudo-aléatoire codé de Golay.

7. Procédé selon la revendication 1, 2 ou 3, caractérisé en ce que le générateur de signal délivre un signal électronique pseudo-aléatoire codé de Barker.

8. Procédé selon une des revendications 1 à 3, caractérisé en ce que le générateur de signal (50) délivre un signal électronique codé de type pseudo-aléatoire de séquence M ; de préférence ayant une durée d'impulsion élémentaire θ comprise entre 0,1 μs et 100 μs, idéalement de valeur 2 à 10 fois la période d'oscillation du transducteur, une période de répétition T comprise entre 1 μs et 10 s.

9. Appareil de thérapie comprenant un dispositif de thérapie proprement dit comprenant au moins un élément transducteur ultrasonore de thérapie (221-230) et un générateur de signal (50) délivrant un signal électronique audit élément transducteur ultrasonore, caractérisé en ce que le générateur de signal délivre un signal électronique dont la fonction d'autocorrélation est de type DIRAC.

10. Appareil selon la revendication 9, caractérisé en ce que le générateur de signal (50) délivre un signal aléatoire de type Gaussien ou Poissonien ou un signal à modulation de fréquence ou de phase.

11. Appareil selon la revendication 10, caractérisé en ce que le générateur de signal (50) délivre un signal pseudo-aléatoire codé à séquence M, de préférence présentant une durée d'impulsion élémentaire θ comprise entre 0,1 μs et 100 μs, idéalement de valeur 2 à 10 fois la période d'oscillation du transducteur, et une période de répétition T comprise entre 1 μs et 10 s.

12. Appareil selon la revendication 9, caractérisé en ce que le générateur de signal (50) délivre un signal pseudo-aléatoire codé de Golay, ou un signal pseudo-aléatoire codé de Barker.

13. Appareil selon l'une des revendications 9 à 12, caractérisé en ce que le dispositif de thérapie (20) se présente sous forme d'une calotte sphérique (22) subdivisée en un réseau annulaire ou en mosaïque d'éléments transducteurs piézoélectriques (221 à 230), chaque élément transducteur (221 à 230) étant relié avantageusement via des amplificateurs individuels tels que 301 à 310, et des lignes à retard (401 à 410), au générateur de signal (50) qui est de préférence lui-même commandé par une centrale de commande (60) qui peut également commander les lignes à retard (401 à 410) en fournissant la valeur de retard nécessaire à chaque ligne pour obtenir une focalisation à distance de focalisation recherchée.

14. Appareil selon l'une des revendications 9 à 13, caractérisé en ce qu'il s'agit d'un appareil de thérapie par ultrasons focalisés pour le traitement de lésions tissulaires au point focal F pour la thérapie du cancer, par exemple de la prostate, du sein, du cerveau.

**Patentansprüche**

1. Elektronisches dynamisches Fokussierungsverfahren mindestens eines piezoelektrischen Ultraschallwandlers für die Therapie (221-230), ohne oder mit minimaler Sekundärfokussierung, umfassend die Inbetriebnahme des Ultraschallwandlerelements durch ein von einem Meßgenerator ausgegebenes elektronisches Signal, gekennzeichnet dadurch, daß man einen Meßgenerator (50) verwendet, welcher ein elektronisches Signal ausgibt, dessen Autokorrelationsfunktion vom Typ DIRAC ist.

2. Verfahren gemäß Anspruch 1, gekennzeichnet dadurch, daß der Meßgenerator (50) ein elektronisches Signal mit Autokorrelationsfunktion vom Typ DIRAC aussendet, das für die Phasen- oder die Frequenzmodulation eines elektronischen Signals verwendet wird, dessen Trägerfrequenz der Nennbetriebsfrequenz des Wandlerelements entspricht.

3. Verfahren gemäß Anspruch 1 oder 2, gekennzeichnet dadurch, daß der Meßgenerator ein elektronisches Signal vom binären Typ mit einer Autokorrelationsfunktion vom Typ DIRAC ausgibt.

4. Verfahren gemäß Anspruch 1, 2 oder 3, gekennzeichnet dadurch, daß der Meßgenerator ein zufälliges elektronisches Signal vom Typ Gauß oder Poisson ausgibt.

5. Verfahren gemäß Anspruch 1, 2 oder 3, gekennzeichnet dadurch, daß der Meßgenerator ein phasen- oder frequenzmoduliertes elektronisches Signal ausgibt.

**6.** Verfahren gemäß Anspruch 1, 2 oder 3, gekennzeichnet dadurch, daß der Meßgenerator ein kodiertes pseudo-zufälliges elektronisches Signal vom Typ Golay ausgibt.

**7.** Verfahren gemäß Anspruch 1, 2 oder 3, gekennzeichnet dadurch. daß der Meßgenerator ein kodiertes pseudo-zufälliges elektronisches Signal vom Typ Barker ausgibt.

**8.** Verfahren gemäß mindestens einem der Ansprüche 1 bis 3, gekennzeichnet dadurch, daß der Meßgenerator (50) ein kodiertes pseudo-zufälliges elektronisches Signal der Sequenz M ausgibt ; bevorzugt mit einer Einzel-Pulsdauer θ zwischen 0,1 μs und 100 μs, im Idealfall mit einem Wert von 2 bis 10 mal der Schwingungsperiode des Wandlers, und mit einer Wiederholungsperiode T zwischen 1 μs und 10 s.

**9.** Therapievorrichtung, umfassend ein Therapie-Gerät an sich, umfassend mindestens ein Ultraschallwandlerelement für die Therapie (221-230) und einen Meßgenerator (50), welcher ein elektronisches Signal zum Ultraschallwandlerelement aussendet, gekennzeichnet dadurch, daß der Meßgenerator ein elektronisches Signal ausgibt, dessen Autokorrelationsfunktion vom Typ DIRAC ist.

**10.** Vorrichtung gemäß Anspruch 9, gekennzeichnet dadurch, daß der Meßgenerator (50) ein zufälliges Signal vom Typ Gauß oder Poisson oder ein Signal mit Frequenz- oder Phasenmodulation ausgibt.

**11.** Vorrichtung gemäß Anspruch 10, gekennzeichnet dadurch, daß der Meßgenerator (50) ein kodiertes pseudo-zufälliges elektronisches Signal der Sequenz M ausgibt, bevorzugt mit einer Einzel-Pulslänge θ zwischen 0,1 μs und 100 μs, im Idealfall mit einem Wert von 2 bis 10 mal der Schwingungsperiode des Wandlers, und mit einer Wiederholungsperiode T zwischen 1 μs und 10 s.

**12.** Vorrichtung gemäß Anspruch 9, gekennzeichnet dadurch, daß der Meßgenerator (50) ein kodiertes pseudo-zufälliges elektronisches Signal vom Typ Golay, oder ein pseudo-zufälliges kodiertes Signal vom Typ Barker ausgibt.

**13.** Vorrichtung gemäß mindestens einem der Ansprüche 9 bis 12, gekennzeichnet dadurch, daß die Therapievorrichtung (20) die Form einer sphärischen Kappe (22) aufweist. welche in ein ring- oder mosaikartiges Netz von piezoelektrischen Wandlern (221 bis 230) unterteilt ist, wobei jedes Wandlerelement (221 bis 230) vorzugsweise über einzelne Verstärker derart wie 301 bis 310 und den Verzögerungsleitungen (401 bis 410) mit dem Meßgenerator (50) verbunden ist, wobei letzterer vorzugsweise selbst über einer Steuerzentrale (60) gesteuert wird, welche ebenfalls in der Lage ist, die Verzögerungsleitungen zu steuern, indem sie den für den Erhalt einer Fokussierung an der gewünschten Brennweite benötigten Verzögerungswert für jede Leitung liefert.

**14.** Vorrichtung gemäß mindestens einem der Ansprüche 9 bis 13, gekennzeichnet dadurch, daß es sich um eine Vorrichtung für die Therapie mit fokussiertem Ultraschall für die Behandlung von Gewebeverletzungen am Brennpunkt F für die Krebstherapie, beispielsweise der Prostata, der Brust, des Gehirns handelt.

## Claims

**1.** A method for dynamic electronic focusing of at least one therapy ultrasound piezoelectric transducer element (221-230), eliminating or minimising secondary focusing, comprising activating the ultrasound transducer element by an electronic signal supplied by a signal generator, characterised in that a signal generator (50) is employed that delivers an electronic signal the self-correlation function of which is of the Dirac type.

**2.** The method of claim 1, characterised in that the signal generator (50) supplied an electronic signal having a Dirac type self-correlation function which is used for phase-or frequency-modulating an electronic signal the carrier frequency of which corresponds to the nominal operating frequency of the transducer element.

**3.** The method of claim 1 or 2, characterised in that the signal generator supplies a binary-type electronic signal having a self-correlation function of the Dirac type.

**4.** The method of claim 1, 2 or 3, characterised in that the signal generator supplies a random Gaussian- or Poissonian-type electronic signal.

**5.** The method of claim 1, 2 or 3, characterised in that the signal generator supplies a frequency- or phase-modulated electronic signal.

6. The method according to claim 1, 2 or 3, characterised in that the signal generator supplies a Golay-coded pseudo-random electronic signal.

7. The method of claim 1, 2 or 3, characterised in that the signal generator supplies a pseudo-random Barker-coded electronic signal.

8. The method according to one of claims 1 to 3, characterised in that the signal generator (50) supplies a pseudo-random type coded electronic signal of a sequence M; preferably having an elementary pulse duration $\Theta$ comprised between 0.1 µs and 100 µs, ideally of a value twice to 10 times the period of oscillation of the transducer, and a repetition period T comprised between 1 µs and 10 seconds.

9. Therapy apparatus comprising an actual therapy device comprising at least one ultrasound therapy transducer element (221- 230) and a signal generator (50) supplying an electronic signal to said ultrasound transducer element, characterised in that the signal generator suppliers an electronic signal the self-correlation function of which is of the Dirac type.

10. The apparatus according to claim 9, characterised in that the signal generator (50) supplies a random signal of the Gaussian or Poissonian type or a frequency-or phase-modulated signal.

11. The apparatus according to claim 10, characterised in that the signal generator (50) supplies a pseudo-random signal coded with a sequence M, preferably having an elementary pulse duration $\Theta$ comprised between 0.1 µs and 100 µs, ideally a value twice to 10 times the period of oscillation of the transducer, and a repetition period T comprised between 1 µs and 10 seconds.

12. The apparatus according to claim 9, characterised in that the signal generator (50) suppliers a pseudo-random signal which is a Golay-coded or a Barker-coded pseudo random signal.

13. The apparatus according to one of claims 9 to 12, characterised in that the therapy device (20) takes the form of a spherical cup (221 sub-divided into an annular array, or a mosaic, of piezoelectric transducer elements (221-230) , each transducer element (221 to 230) being advantageously connected, via individual amplifiers such as 301 to 310 and delay lines (401 - 410), to the signal generator (50) which is preferably itself commanded by a control unit (60) which can also command the delay lines (401 to 410) by supplying the delay value needed by each line in order to obtain a distant focusing of the desired focus.

14. The apparatus according to one of claims 9 to 13, characterised in that it is a focused ultrasound therapy apparatus for treating tissue lesions at the focal point F for cancer therapy, for example of the prostate, breast or brain.

FIG.1

FIG.2  (ART ANTERIEUR)

FIG. 3  (ART ANTERIEUR)

FIG. 4 (ART ANTERIEUR)

FIG.5 (INVENTION)

FIG.7 (INVENTION)

FIG.7 (INVENTION)

FIG. 8  (INVENTION)